# EUROPEAN PATENT SPECIFICATION

(11) **EP 3 541 337 B1**
(45) Date of publication and mention of the grant of the patent: **04.11.2020**
(21) Application number: 16795396.7
(22) Date of filing: 16.11.2016
(51) Int. Cl.: A61F 13/15, B05C 1/04, B05C 1/08, B05D 1/28

(54) **APPARATUS AND METHOD FOR COATING A WEB OF ABSORBENT SUBSTRATE**
VORRICHTUNG UND VERFAHREN ZUR BESCHICHTUNG EINER BAHN EINES ABSORBIERENDEN SUBSTRATS
APPAREIL ET PROCÉDÉ DE REVÊTEMENT D'UNE BANDE DE SUBSTRAT ABSORBANT

(43) Date of publication of application: 25.09.2019
(73) Proprietor: ESSITY HYGIENE AND HEALTH AKTIEBOLAG, 405 03 Göteborg (SE)
(72) Inventor: RUPPEL, Rémy, 68320 Durrenentzen (FR); BARREDO, Donald, 68040 Ingersheim (FR)
(74) Representative: Nederlandsch Octrooibureau
(86) International application number: PCT/EP2016/077909
(87) International publication number: WO 2018/091087

(56) References cited:
- EP-A1- 2 292 199
- US-A- 4 282 275
- US-A1- 2006 147 637

## Description

### TECHNICAL FIELD

An aspect relates to an apparatus for coating a web of absorbent substrate. Another aspect relates to a method for coating a web of absorbent substrate. Still another aspect relates to a converting machine that includes such an apparatus for coating a web of absorbent substrate. Such an absorbent substrate may be converted into absorbent sheet products that have a particular, though non-exclusive, use as sanitary or domestic purposes. As an example, such absorbent sheet products may be used as toilet paper in restrooms. Other uses such as napkins, towels, kitchen towels, hand towels, toilet papers, wipes, facial tissues, bath tissues, etc... are possible.

### BACKGROUND

The web of absorbent substrate may be a web of tissue paper that is obtained by a Conventional Wet Press or Through Air Drying manufacturing method or other manufacturing method. A tissue paper relates to an absorbent paper based on cellulose fibers which is also called tissue paper base sheet in this field of technology. A typical absorbent paper has a low basis weight, in the range from 10 to 60 g/m², or 30 to 50 g/m².

The web of absorbent substrate may also be a web of nonwoven fabric that is obtained by an air-laid manufacturing method or spun-laid manufacturing method or other manufacturing method. A nonwoven fabric including cellulosic fibers relates to an absorbent paper which is also called nonwoven or web made of fibers like air-laid web in this field of technology. A typical absorbent paper has a basis weight, in the range from 20 to 300 g/m², or 40 to 60 g/m².

A web of absorbent substrate is submitted to various converting steps before being wound as a log. For example, one converting step includes providing a ply of absorbent substrate with defined aesthetical pattern, another converting step includes applying coating material to a ply of absorbent substrate, still another converting step includes associating various plies of absorbent substrate together, still another converting step includes providing the web with regularly spaced transverse perforation/weakening lines, etc...

The document US 4,814,204 describes an apparatus for applying spaced apart discontinuous stripes of a fluid to the surface of a moving web. The apparatus includes a rotating applicator roll partially immersed in a bath of the fluid. A notched doctor blade in contact with the surface of the roll removes fluid from the surface of the roll except in the region of the notches to form spaced apart circumferential beads of the fluid on the surface of the roll. The moving web partially wraps the applicator roll where the beads are formed, but is travelling between 20 to 200 times the speed of the surface of the applicator roll, which results in a relatively even distribution of the fluid, in the form of a discontinuous stripe, on the surface of the web. US4282275 discloses a substrate web apparatus for applying a coating material on a web including a support device to support the web, a dosing and transfer device arranged to cooperate with a material storing and transferring unit such as to continuously coat a surface of the dosing and transfer device with an uniform quantity of coating material.

However, such a doctored single kiss roll applicator is not adapted for coating a moving web in a discontinuous manner, namely at specific locations of the web, or in an intermittent manner, namely at defined time of the web manufacturing process.

Further, it is also desired to take into account the current trend of industrial manufacturing speed of converting process (up to 1.200 m/min) generating constraints on the web that may result in the web breaking-up.

Thus, there is a need to improve the coating of a web of absorbent substrate in a converting machine. Further, this should be obtained in an economical or a cost-effective manner.

### SUMMARY

It is desirable to propose an apparatus and/or method for coating a web of absorbent substrate that overcomes the drawbacks of the prior art coating apparatus and/or method. In particular, it is desirable to enable discontinuous or intermittent coating of a moving web of absorbent substrate. More particularly, it is desirable to solve this issue even for web of absorbent substrate running at an industrial manufacturing speed in a converting machine (e.g. up to 1.200 m/min).

According to one aspect, there is provided an absorbent substrate web coating apparatus for applying a coating material on a web of absorbent substrate over a defined length including:
- a support device arranged to support the web of absorbent substrate running at a speed ranging from around 200 m/min to around 1.200°m/min, and
- a dosing and transfer device arranged to cooperate with a coating material storing and transferring unit such as to continuously coat a surface of the dosing and transfer device with a uniform quantity of coating material. The absorbent substrate web coating apparatus further includes an oscillating mechanism arranged to oscillate the dosing and transfer device or the support device from an engaged position to a remote position, and the dosing and transfer device is arranged relatively to the support device such that the transfer device applies the coating material to the web of absorbent substrate at a contacting location where the web of absorbent material contacts the support device and the dosing and transfer device only in the engaged position.

The support device may be a support roller or a support plate. The dosing and transfer device may include a transfer roller.

The dosing and transfer device may include a dosing roller and a transfer roller, the dosing roller being arranged to cooperate with the coating material storing and transferring unit such as to continuously coat the surface of the dosing roller with a uniform quantity of coating material, and the transfer roller being coupled to the dosing roller such as to transfer the coating material from the dosing roller to the transfer roller.

The oscillating mechanism may include a rotating mechanism arranged to cause the transfer roller to rotate around a circumference of the dosing roller over an angular sector defined by the engaged and remote position and centered on a rotary shaft of the dosing roller.

The oscillating mechanism may include a translating mechanism arranged to cause the dosing roller and the transfer roller to translate together over a distance defined by the engaged and remote position.

An additional transfer roller may be positioned between the support roller and the transfer roller and permanently contacting the support roller whatever the position of the transfer roller.

The support roller may be an eccentric roller.

The oscillating mechanism may include another rotating mechanism including an oscillating arm coupled at one end to a rotary shaft of the support roller, and at another end to a pivot, the rotating mechanism being arranged to cause the support roller to rotate over an angular sector defined by the engaged and remote position and centered on the pivot.

The oscillating mechanism may include another translating mechanism arranged to cause the support roller to translate over a distance defined by the engaged and remote position.

The coating material storing and transferring unit may be chosen among the group of a chamber doctor blade, a spraying unit, and a slot dye.

According to another aspect, there is provided a converting machine including an embossing unit and/or a rewinding unit, wherein the converting machine further includes an absorbent substrate web coating apparatus according to an embodiment of the invention located proximate to the embossing unit and/or located proximate to and upstream of the rewinding unit.

According to a further aspect, there is provided a use of the converting machine described above for manufacturing absorbent sheet products chosen among the group of napkins, towels, kitchen towels, hand towels, toilet papers, wipes and facial tissues.

According to still a further aspect, there is provided an absorbent substrate web coating method for applying a coating material on a web of absorbent substrate over a defined length including:
- conveying the web of absorbent substrate at a speed ranging from around 200 m/min to around 1.200°m/min and supporting the conveyed web of absorbent substrate by means of a support device,
- continuously coating a surface of a dosing and transfer device with a uniform quantity of coating material by means of a coating material storing and transferring unit cooperating with the dosing and transfer device
- oscillating the dosing and transfer device or the support device from a engaged position to a remote position, and
- applying the coating material to the web of absorbent substrate at a contacting location where the web of absorbent substrate contacts the support device and the dosing and transfer device only in the engaged position.

The dosing and transfer device may include a transfer roller. The oscillating step may include rotating the transfer roller from an engaged position to a remote position over an angular sector defined by the engaged and remote position.

The oscillating step may include translating the transfer roller over a distance defined by the engaged and remote position.

The support device may be a support roller. The oscillating step may include eccentrically rotating the support roller, the engaged position corresponding to a defined angular sector of the support roller.

The oscillating step may include rotating the support roller over an angular sector defined by the engaged position and remote position.

The oscillating step may include translating the support roller over a distance defined by the engaged position and remote position.

With embodiments of the invention, it is possible to apply coating material to the web of absorbent substrate in a discontinuous or intermittent fashion. Thus, the coating of the web of absorbent substrate could be applied only where it is necessary.

At the same time, with embodiments of the invention, the risk of the web breaking-up when the coating operation begins is avoided, or at least significantly reduced.

Other advantages will become apparent from the hereinafter description of particular embodiments of the invention.

### BRIEF DESCRIPTION OF THE DRAWINGS

Embodiments of the present invention are illustrated by way of examples and not limited to the accompanying drawings, in which like references indicate similar elements:
- FIGS. 1 and 2 are side views schematically illustrating a first embodiment of an apparatus for coating a web of absorbent substrate in an engaged position and in a remote position, respectively, said first embodiment including a chamber doctor blade and a rotating mechanism;
- FIGS. 3 and 4 are side views schematically illustrating a second embodiment of an apparatus for coating a web of absorbent substrate in an engaged position and in a remote position, respectively, said second embodiment including a chamber doctor blade, a rotating mechanism and an additional transfer roller;
- FIGS. 5 and 6 are side views schematically illustrating a third embodiment of an apparatus for coating a web of absorbent substrate in an engaged position and in a remote position, respectively, said third embodiment including a chamber doctor blade and a translating mechanism;
- FIGS. 7 and 8 are side views schematically illustrating a fourth embodiment of an apparatus for coating a web of absorbent substrate in an engaged position and in a remote position, respectively, said fourth embodiment including a chamber doctor blade, a translating mechanism and an additional transfer roller;
- FIGS. 9, 10, 11 and 12 are side views schematically illustrating alternatives to the first and second embodiments using a spraying unit and a slot dye, respectively;
- FIGS. 13, 14, 15 and 16 are side views schematically illustrating alternatives to the third and fourth embodiments using a spraying unit and a slot dye, respectively;
- FIGS. 17 and 18 are side views schematically illustrating a fifth embodiment of an apparatus for coating a web of absorbent substrate in an engaged position and in a remote position, respectively, said fifth embodiment including a support roller having the shape of a cam;
- FIGS. 19 and 20 are side views schematically illustrating a sixth embodiment of an apparatus for coating a web of absorbent substrate in an engaged position and in a remote position, respectively, said sixth embodiment including a support roller coupled to a rotating mechanism;
- FIGS. 21 and 22 are side views schematically illustrating a seventh embodiment of an apparatus for coating a web of absorbent substrate in an engaged position and in a remote position, respectively, said seventh embodiment including a translating support roller; and
- FIG. 23 is a side view schematically illustrating a converting machine/line including an apparatus for coating a web of absorbent substrate.

### DETAILED DESCRIPTION OF PARTICULAR EMBODIMENTS

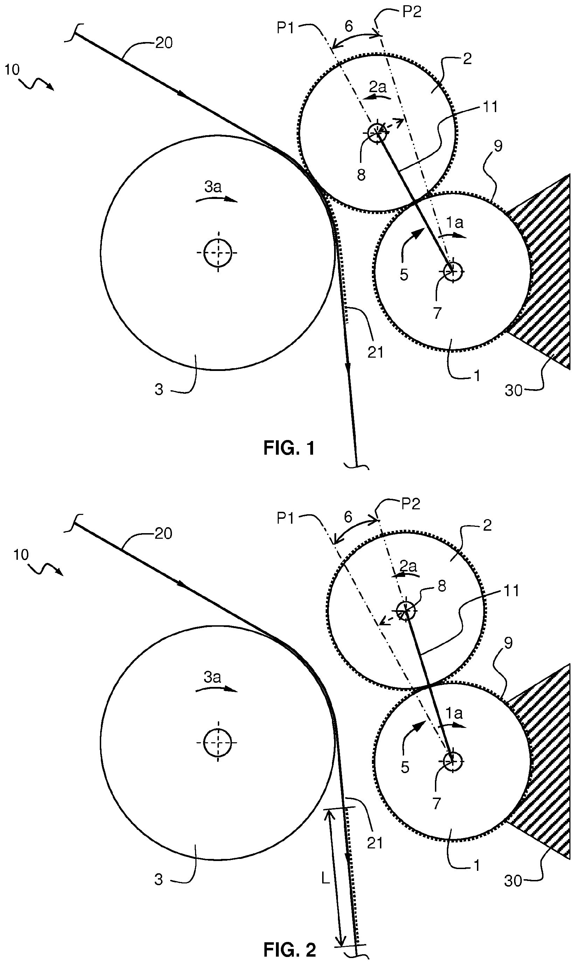

A first embodiment of an apparatus for coating a web of absorbent substrate is schematically illustrated in FIGS. 1 and 2. FIG. 1 is a side view showing the absorbent substrate web coating apparatus 10 in an engaged position P1. FIG. 2 is a side view showing the absorbent substrate web coating apparatus 10 in a remote position P2. The absorbent substrate web coating apparatus 10 according to the first embodiment is based on a chamber doctor blade and a rotating mechanism that will be described in details hereinafter. The absorbent substrate web coating apparatus 10 is used to apply a coating material 21 on the web of absorbent substrate 20 over a defined length L. The absorbent substrate web coating apparatus 10 includes a dosing roller 1, a transfer roller 2, a support roller 3, a coating material storing and transferring unit realized under the form of a chamber doctor blade 30 and an oscillating mechanism 5.

The support roller 3 supports the web of absorbent substrate 20 running at a speed ranging from around 200 m/min to around 1.200°m/min. The support roller 3 is driven to rotate in the direction indicated by the arrow 3a by an appropriate motor and transmission mechanism (not shown). The web of absorbent substrate 20 may be further supported by guide rollers (not shown) upstream and downstream the support roller 3 so that the web of absorbent substrate 20 partially wraps the support roller 3. The web of absorbent substrate 20 may come from an unwinding unit (not shown) from a large parent roll. The web of absorbent substrate 20 may be submitted to a converting process including various steps, and may be ultimately rewind as retailed sized rolls having particular characteristics. For example, the converting process converts a large parent web (e.g. having a strip width from around 1,80 m to around 7 m) into retail sized rolls like bathroom tissue rolls or paper towels rolls (e.g. having a strip width from around 8 cm to around 40 cm).

The dosing roller 1 cooperates with the chamber doctor blade 30 (i.e. the coating material storing and transferring unit). Due to the rotation of the dosing roller 1, a circumferential surface 9 of the dosing roller 1 is continuously coat with a uniform quantity of coating material 21. The transfer roller 2 is coupled to the dosing roller 1 in the sense that there is a contacting point between both rollers. The coating material is transferred from the dosing roller 1 to the transfer roller 2 at this contacting point. The dosing roller 1 and the transfer roller 2 are driven to rotate in the respective directions indicated by the arrows 1a and 2a by an appropriate motor and transmission mechanism (not shown). The dosing roller 1 and the transfer roller 2 are rotating in counter-directions. The support roller 3 and the transfer roller 2 are also rotating in counter-directions. The support roller 3 and the transfer roller 2 are rotating at a speed close to the linear speed of the web of absorbent substrate 20.

A chamber doctor blade 30 is a known device to apply a defined quantity of coating material in a uniform manner on a surface of a roller. The dosing roller 1 is rotating in a manner partially immersed in a tank of the chamber doctor blade 30 containing a coating material. The doctor blade or scraping blade wipe off excess coating material from the surface 9 of the dosing roller 1 and smooth out the film of coating material adhering to the surface 9 of the dosing roller 1 before the film is rotated into contact with the transfer roller 2. The excess coating material is recycled back to the container of the chamber doctor blade 30. The coating material may be a liquid composition or a suspension. For example, the coating material includes water, or a water based composition, or a polymer such as a polyether polyol, like polyethylene glycol or polypropylene glycol.

The oscillating mechanism 5 is arranged to oscillate by rotation of the transfer roller 2 from the engaged position P1 (FIG. 1) to the remote position P2 (FIG. 2).

The engaged position P1 (FIG. 1) corresponds to a position where the web of absorbent material 20 contacts both the support roller 3 and the transfer roller 2. In practice, the web of absorbent material 20 travels through a nip between the support roller 3 and the transfer roller 2. Said nip is sufficiently narrow so that the web contacts both the support roller 3 on one side and the transfer roller 2 on the other side. This position defined a contacting location where the coating material 21 can be applied onto the moving web of absorbent material 20.

The remote position P2 (FIG. 2) corresponds to a position where the transfer roller 2 is away from the support roller 3. In this position, the web of absorbent material 20 travels over the support roller 3 and is not in contact with the transfer roller 2. When oscillating from the engaged position P1 to the remote position P2, the transfer roller 2 is swept over an angular sector 6 corresponding to various positions. In the present description, the remote position also corresponds to these various positions between the engaged position P1 and the remote position P2 where the transfer roller 2 is away from the support roller 3 and from the moving web of absorbent material 20 such that the coating material 21 cannot be applied to the moving web of absorbent material 20.

In summary, it is only when the transfer roller 2 is in the engaged position (FIG. 1) relatively to the support roller 3 that the transfer roller 2 applies the coating material 21 to the web of absorbent substrate 20 at the contacting location. In the engaged position P1, the sequence of contact between the chamber doctor blade 30, the dosing roller 1, the transfer roller 2, the running web of absorbent substrate 20 and the support roller 3 results in applying sufficiently and uniformly the coating material 21 to the web 20. It is the duration of the engaged position P1 that determines the coating length L where the coating material 21 is applied to the web of absorbent substrate 20. For example, when the web 20 travels at a speed of 600 m/min or 10 m/s, a duration of the engaged position around 1/10^{th} of second corresponds to a coating length L of 1 m. It is the synchronization of the oscillation movement that determines the application of the coating material with respect to particular positions along web portions (a web potions may define the total length of the web available in a retailed sized roll), and in particular with respect to the first windings defining the hole of the subsequently formed retailed sized roll, or the leading edges and/or trailing edges of the subsequently formed retailed sized rolls. Also, by adjusting the synchronization of the oscillation movement, it is possible to provide each web portions with defined stamping pattern of coating material (e.g. series of stripes of coating material).

In the present embodiment, the oscillating mechanism is a rotating mechanism 5, for example an oscillating arm 11 coupled to a driving means (e.g. a motor not shown). The oscillating arm 11 is coupled at one end to a rotary shaft 7 of the dosing roller 1, and at another end to another rotary shaft 8 of the transfer roller 2. The oscillating arm 11 is driven to oscillate about the rotary shaft 7 of the dosing roller 1. The transfer roller 2 is driven to move along an oscillating orbit by the operation of the driving means and the oscillating arm 11. The driving means further limits the axial movement of the oscillating arm 11 between selected limits defining the angular sector 6. Thus, the transfer roller 2 rotates back and forth around a circumference 9 of the dosing roller 1 over the angular sector 6 defined by the engaged and remote positions P1 and P2 and centered on the rotary shaft 7 of the dosing roller 1. The rotary shaft 7 of the dosing roller 1 is coincident with an axle of the dosing roller.

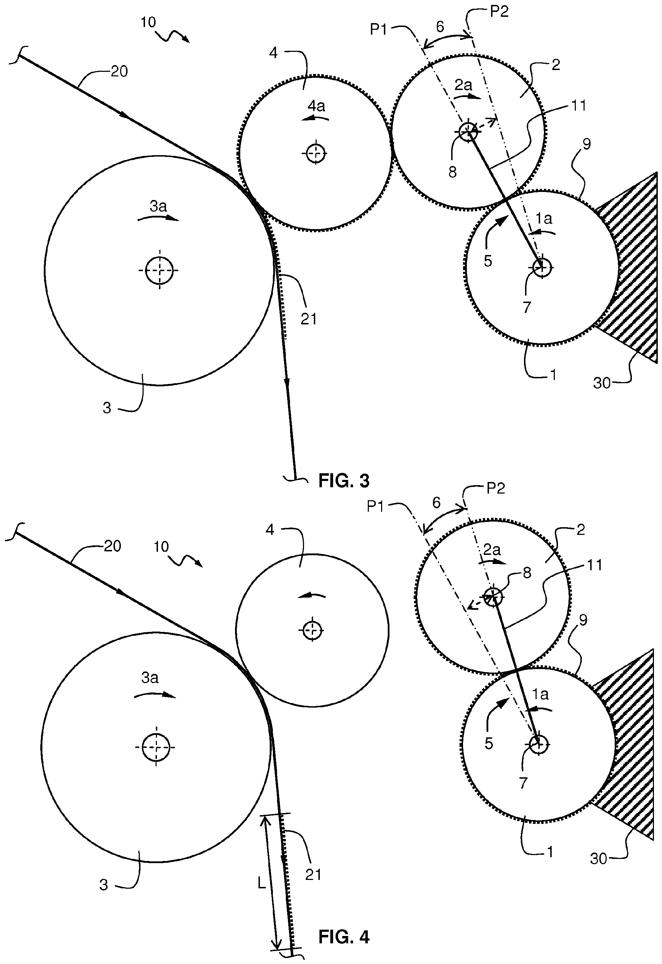

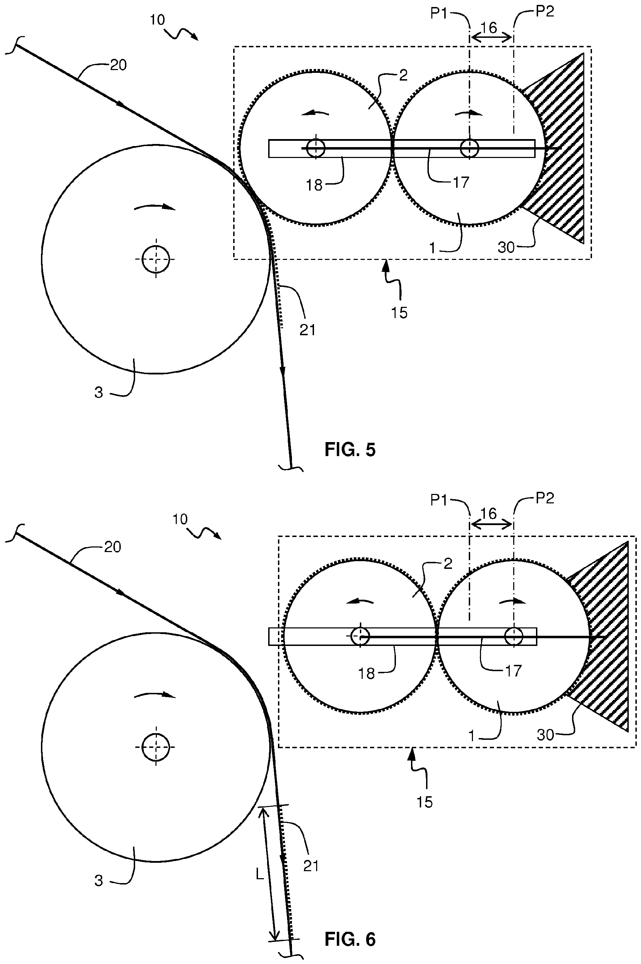

A second embodiment of an apparatus for coating a web of absorbent substrate is schematically illustrated in FIGS. 3 and 4. FIG. 3 is a side view showing the absorbent substrate web coating apparatus 10 in an engaged position P1. FIG. 6 is a side view showing the absorbent substrate web coating apparatus 10 in a remote position P2. The absorbent substrate web coating apparatus 10 according to the second embodiment differs from the first embodiment in that an additional transfer roller 4 is positioned between the support roller 3 and the transfer roller 2. The additional transfer roller 4 is permanently contacting the travelling web 20 and the support roller 3 whatever the position of the transfer roller 2. The support roller 3 and the additional transfer roller 4 are driven to rotate in the respective directions indicated by the arrows 3a and 4a by an appropriate motor and transmission mechanism (not shown). The dosing roller 1 and the transfer roller 2 are rotating in counter-directions. Similarly to the first embodiment, it is only when the transfer roller 2 is in the engaged position (FIG. 1) that a contact is established with the additional transfer roller 4. In the engaged position, the transfer roller 2 applies the coating material 21 to the web of absorbent substrate 20 via the additional transfer roller 4 at the contacting location. In the engaged position P1, the sequence of contact between the chamber doctor blade 30, the dosing roller 1, the transfer roller 2, the additional transfer roller 4 and the running web of absorbent substrate 20 and the support roller 3 results in applying sufficiently and uniformly the coating material 21 to the web 20. It is the duration of the engaged position P1 that determines the coating length L where the coating material 21 is applied to the web of absorbent substrate 20. This embodiment where the web of absorbent substrate 20 is travelling between the support roller 3 and the additional transfer roller 4 enables reducing the risk of the web being broken when the transfer roller 2 comes into engagement with the additional transfer roller 4 by damping the effect of transition between the remote position and the engaged position.

A third embodiment of an apparatus for coating a web of absorbent substrate is schematically illustrated in FIGS. 5 and 6. FIG. 5 is a side view showing the absorbent substrate web coating apparatus 10 in an engaged position P1. FIG. 6 is a side view showing the absorbent substrate web coating apparatus 10 in a remote position P2. The absorbent substrate web coating apparatus 10 according to the third embodiment is based on a chamber doctor blade and differs from the first embodiment in that the rotating mechanism is replaced by a translating mechanism 15.

In the present embodiment, the oscillating mechanism is a translating mechanism 15 is arranged to oscillate by translation the transfer roller 2 together with the dosing roller 1 and the chamber doctor blade 30 over a defined distance 16 from the engaged position P1 (FIG. 5) to the remote position P2 (FIG. 6). The distance 16 is defined by the extreme engaged and remote positions P1 and P2, respectively.

The engaged position P1 (FIG. 5) corresponds to a position where the web of absorbent material 20 contacts both the support roller 3 and the transfer roller 2. In practice, the web of absorbent material 20 travels through a nip between the support roller 3 and the transfer roller 2. Said nip is sufficiently narrow so that the web contacts both the support roller 3 on one side and the transfer roller 2 on the other side. This position defined a contacting location where the coating material 21 can be applied onto the moving web of absorbent material 20.

The remote position P2 (FIG. 6) corresponds to a position where the transfer roller 2, the dosing roller 3 and the chamber doctor blade 30 are away from the support roller 3. In this position, the web of absorbent material 20 travels over the support roller 3 and is not in contact with the transfer roller 2. When oscillating from the engaged position P1 to the remote position P2, the transfer roller 2, the dosing roller 3 and the chamber doctor blade 30 are translated over a defined distance 16 corresponding to various positions. In the present description, the remote position also corresponds to these various positions between the engaged position P1 and the remote position P2 where the transfer roller 2, the dosing roller 3 and the chamber doctor blade 30 are away from the support roller 3 and from the moving web of absorbent material 20 such that the coating material 21 cannot be applied to the moving web of absorbent material 20.

In summary, it is only when the transfer roller 2, the dosing roller 3 and the chamber doctor blade 30 are in the engaged position (FIG. 5) relatively to the support roller 3 that the transfer roller 2 applies the coating material 21 to the web of absorbent substrate 20 at the contacting location. In the engaged position P1, the sequence of contact between the chamber doctor blade 30, the dosing roller 1, the transfer roller 2, the running web of absorbent substrate 20 and the support roller 3 results in applying sufficiently and uniformly the coating material 21 to the web 20. Similarly to the other embodiments, the duration of the engaged position P1 determines the coating length L where the coating material 21 is applied to the web of absorbent substrate 20. Further, the synchronization of the translation movement determines the application of the coating material with respect to particular positions along the web, and in particular with respect to leading edge and/or trailing edge of web portions that will subsequently forms retailed sized rolls.

In the present embodiment, the oscillating mechanism is a translating mechanism 15, for example a supporting piece 17 moving in a guide 18 coupled to a driving means (e.g. a motor not shown). The supporting piece 17 supports the chamber doctor blade 30, the dosing roller 1 and the transfer roller 2 such that their respective positions are defined and fixed. The supporting piece 17 is driven to oscillate by translation along the guide 18. The supporting piece 17 is driven to move along the defined distance 16 by the operation of the driving means. The driving means further limits the translation movement of the supporting piece 17 between selected limits defining the distance 16. Thus, the chamber doctor blade 30, the dosing roller 1 and the transfer roller 2 translate back and forth over the distance 16 defined by the engaged and remote positions P1 and P2.

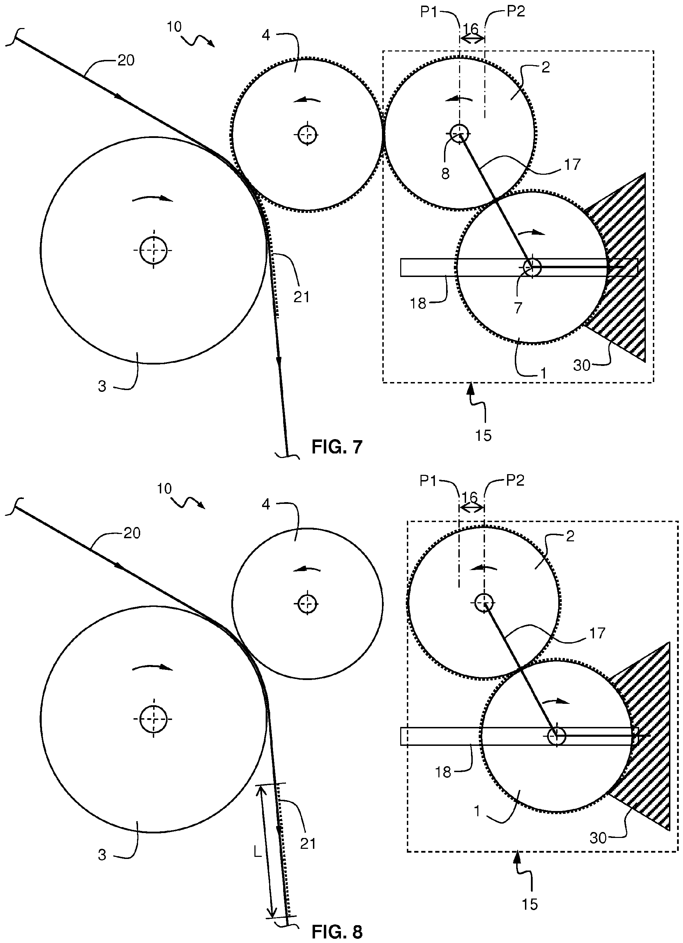

A fourth embodiment of an apparatus for coating a web of absorbent substrate is schematically illustrated in FIGS. 7 and 8. FIG. 7 is a side view showing the absorbent substrate web coating apparatus 10 in an engaged position P1. FIG. 8 is a side view showing the absorbent substrate web coating apparatus 10 in a remote position P2. The absorbent substrate web coating apparatus 10 according to the fourth embodiment is based on a chamber doctor blade and differs from the third embodiment in that an additional transfer roller 4 is positioned between the support roller 3 and the transfer roller 2. The additional transfer roller 4 is permanently contacting the travelling web 20 and the support roller 3 whatever the position of the transfer roller 2, the dosing roller 1 and the chamber doctor blade 30. The function and operation of the additional transfer roller 4 is similar to the second embodiment. As previously described in relation with the third embodiment, the translating mechanism 15 is arranged to oscillate by translation the transfer roller 2 together with the dosing roller 1 and the chamber doctor blade 30 over the defined distance 16 from the engaged position P1 (FIG. 7) to the remote position P2 (FIG. 8). The distance 16 is defined by the extreme engaged and remote positions P1 and P2, respectively. While in the third embodiment (see FIGS. 6 and 7), the transfer roller 2 is aligned with the dosing roller 1 and the chamber doctor blade 30, in the fourth embodiment the axle 8 of the transfer roller 2 is offset from the aligned dosing roller 1 and chamber doctor blade 30. In this case, the supporting piece 17 may be designed as having two sections appropriately inclined with respect to each other.

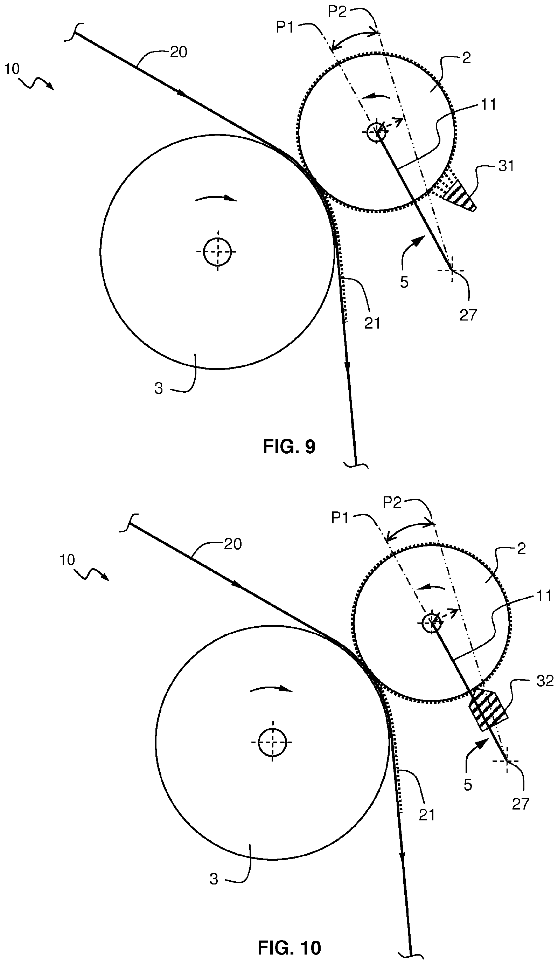

FIG. 9 is a side view schematically illustrating an alternative to the first embodiment (i.e. the embodiment of FIGS. 1 and 2) wherein the chamber doctor blade 30 and dosing roller 1 have been replaced by a spraying unit 31, the oscillating arm 11 of the rotating mechanism 5 being driven to oscillate about a pivot 27. FIG. 10 is a side view schematically illustrating an alternative to the first embodiment wherein the chamber doctor blade 30 and dosing roller 1 have been replaced by a slot dye 32. Otherwise, the operations of these alternative embodiments are similar to the first embodiment.

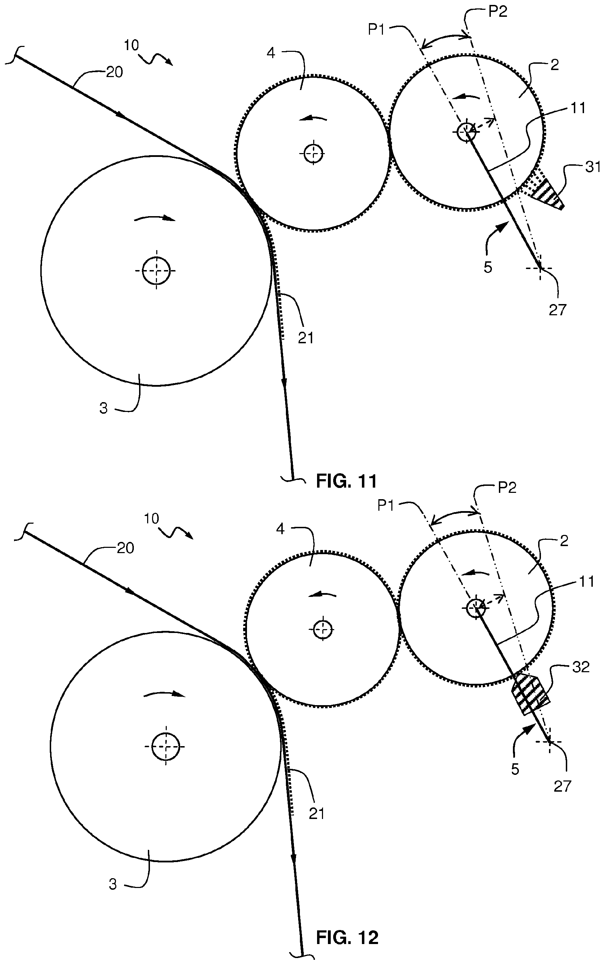

FIG. 11 is a side view schematically illustrating an alternative to the second embodiment (i.e. the embodiment of FIGS. 3 and 4) wherein the chamber doctor blade 30 and dosing roller 1 have been replaced by a spraying unit 31, the oscillating arm 11 of the rotating mechanism 5 being driven to oscillate about a pivot 27. FIG. 12 is a side view schematically illustrating an alternative to the second embodiment wherein the chamber doctor blade 30 and dosing roller 1 have been replaced by a slot dye 32. Otherwise, the operations of these alternative embodiments are similar to the second embodiment.

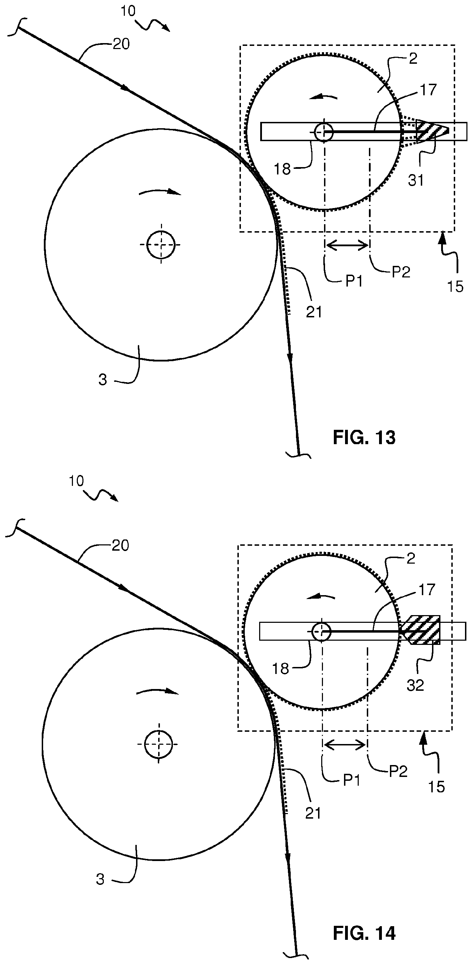

FIG. 13 is a side view schematically illustrating an alternative to the third embodiment (i.e. the embodiment of FIGS. 5 and 6) wherein the chamber doctor blade 30 and dosing roller 1 have been replaced by a spraying unit 31. FIG. 14 is a side view schematically illustrating an alternative to the third embodiment wherein the chamber doctor blade 30 and dosing roller 1 have been replaced by a slot dye 32. Otherwise, the operations of these alternative embodiments are similar to the third embodiment.

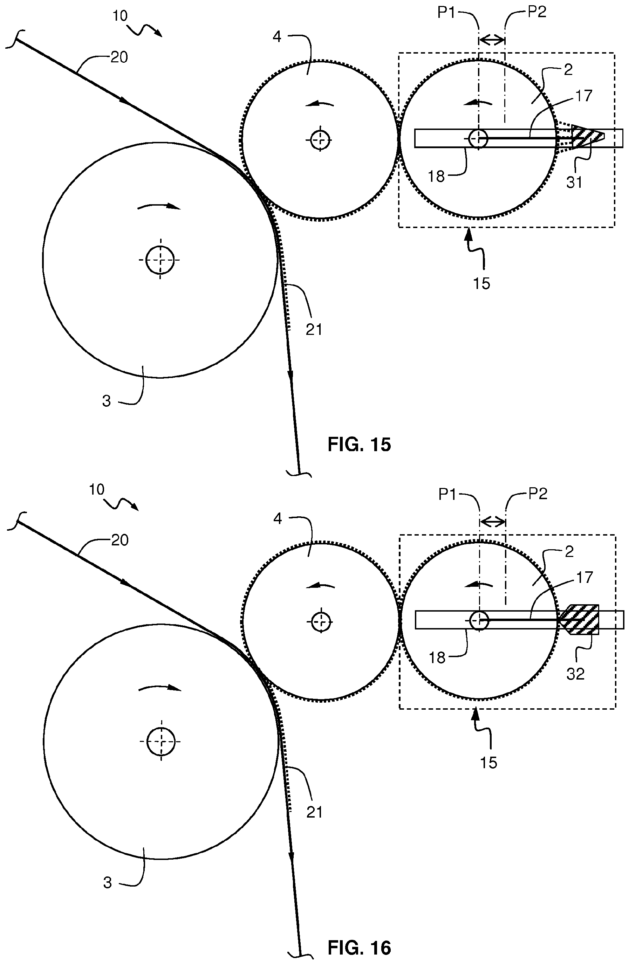

FIG. 15 is a side view schematically illustrating an alternative to the fourth embodiment (i.e. the embodiment of FIGS. 7 and 8) wherein the chamber doctor blade 30 and dosing roller 1 have been replaced by a spraying unit 31. FIG. 16 is a side view schematically illustrating an alternative to the fourth embodiment wherein the chamber doctor blade 30 and dosing roller 1 have been replaced by a slot dye 32. Otherwise, the operations of these alternative embodiments are similar to the fourth embodiment.

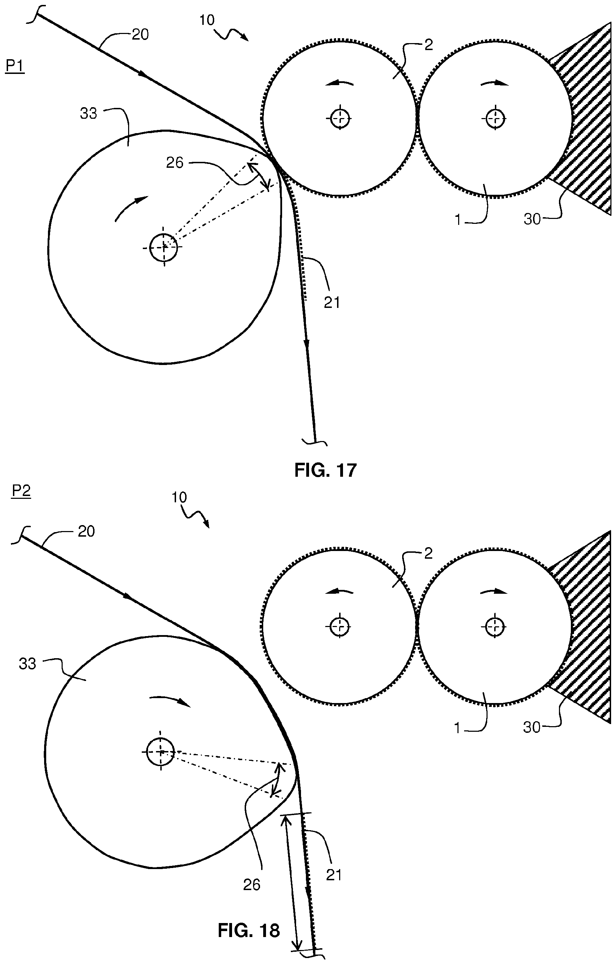

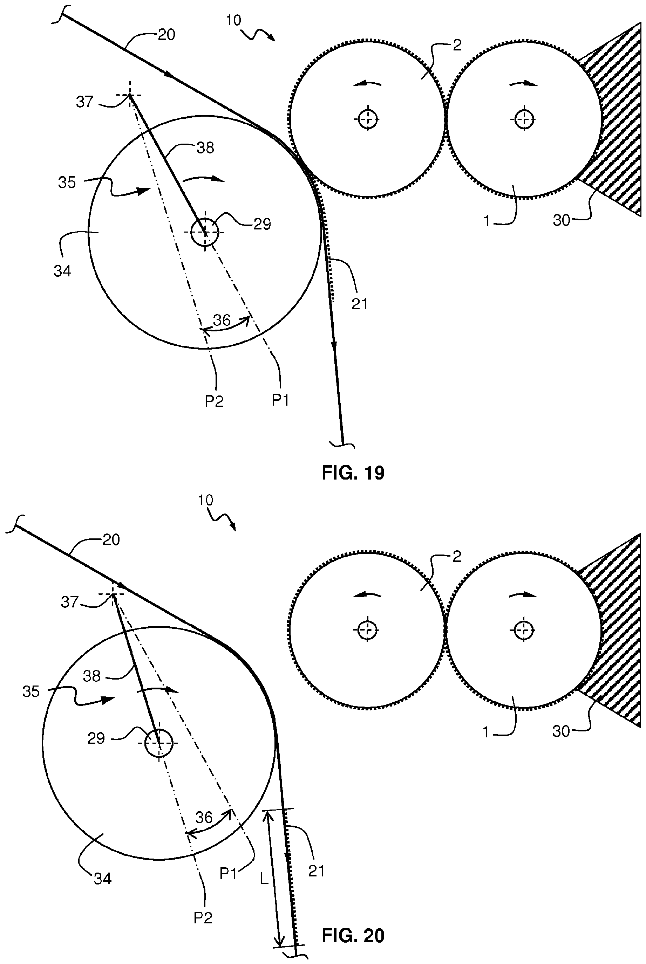

A fifth embodiment of an apparatus for coating a web of absorbent substrate is schematically illustrated in FIGS. 17 and 18. FIG. 17 is a side view showing the absorbent substrate web coating apparatus 10 in an engaged position P1. FIG. 18 is a side view showing the absorbent substrate web coating apparatus 10 in a remote position P2. The absorbent substrate web coating apparatus 10 according to the fifth embodiment is based on a chamber doctor blade 30 and differs from the hereinbefore described embodiment in that the chamber doctor blade 30, the dosing roller 1 and the transfer roller 2 are fixed while the support roller is moving the web of absorbent material 20 towards the transfer roller 2. According to fifth embodiment, the cylindrical support roller 3 is replaced by an eccentric support roller 33, for example a cam like roller. However, any support roller with an irregular shape would be satisfactory to achieve the function of oscillating the web of absorbent material 20 from the engaged position P1 (FIG. 19) to the remote position P2 (FIG. 20). In the present embodiment, the oscillating mechanism is the shape of the eccentric support roller 33 itself. It is only when the web of absorbent material 20 wraps the eccentric support roller 33 as indicated by the contacting angle 26 during the rotation of the eccentric support roller 33 that the web of absorbent material 20 is pushed towards the transfer roller 2 and contacts the transfer roller 2. When the eccentric support roller 33 is in the engaged position P1 relatively to the transfer roller 2, the transfer roller 2 applies the coating material 21 to the web of absorbent substrate 20 at the contacting location. In the engaged position P1, the sequence of contact between the chamber doctor blade 30, the dosing roller 1, the transfer roller 2, the running web of absorbent substrate 20 and the eccentric support roller 33 results in applying sufficiently and uniformly the coating material 21 to the web 20. It is the duration of the engaged position P1 that determines the coating length L where the coating material 21 is applied to the web of absorbent substrate 20. Alternatively, it is also possible to control the coating length L by designing the shape of the eccentric support roller 33 and in particular defining the contacting angle 26. In the fifth embodiment, the chamber doctor blade 30 may be replaced by a spraying unit or a slot dye (not shown).

A sixth embodiment of an apparatus for coating a web of absorbent substrate is schematically illustrated in FIGS. 19 and 20. FIG. 19 is a side view showing the absorbent substrate web coating apparatus 10 in an engaged position P1. FIG. 20 is a side view showing the absorbent substrate web coating apparatus 10 in a remote position P2. The absorbent substrate web coating apparatus 10 according to the sixth embodiment is based on a chamber doctor blade 30 and differs from the hereinbefore described first to fourth embodiments in that the chamber doctor blade 30, the dosing roller 1 and the transfer roller 2 are fixed while the support roller is moving the web of absorbent material 20 towards the transfer roller 2. According to sixth embodiment, the support roller is 3 is replaced by an oscillating support roller 34. In the present embodiment, the oscillating mechanism is a rotating mechanism 35, for example an oscillating arm 38 coupled to a driving means (e.g. a motor not shown). The oscillating arm 38 is coupled at one end to a rotary shaft 29 of the support roller 34, and at another end to a pivot 37. The oscillating arm 38 is driven to oscillate about the pivot 37. The support roller 34 is driven to move along an oscillating orbit by the operation of the driving means and the oscillating arm 38. The driving means further limits the axial movement of the oscillating arm 38 between selected limits defining an angular sector 36. Thus, the support roller 34 rotates back and forth over the angular sector 36 defined by the engaged and remote positions P1 and P2 and centered on the rotary shaft 29 of the support roller 34.

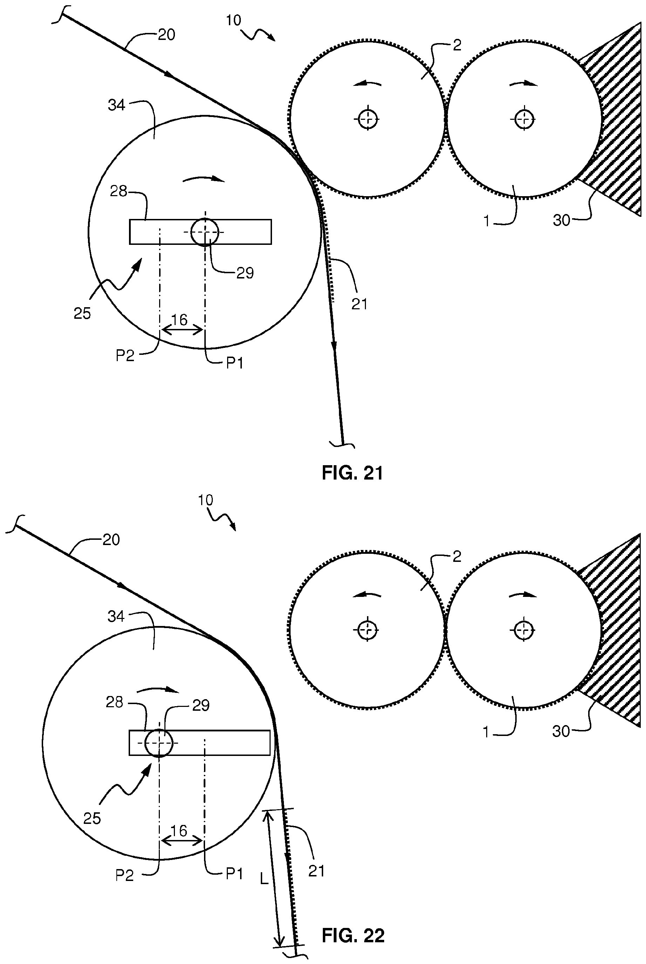

A seventh embodiment of an apparatus for coating a web of absorbent substrate is schematically illustrated in FIGS. 21 and 22. FIG. 21 is a side view showing the absorbent substrate web coating apparatus 10 in an engaged position P1. FIG. 22 is a side view showing the absorbent substrate web coating apparatus 10 in a remote position P2. The absorbent substrate web coating apparatus 10 according to the seventh embodiment is based on a chamber doctor blade 30 and differs from the hereinbefore described first to fourth embodiments in that the chamber doctor blade 30, the dosing roller 1 and the transfer roller 2 are fixed while the support roller is moving the web of absorbent material 20 towards the transfer roller 2. According to the seventh embodiment, the support roller is 3 is replaced by a oscillating support roller 34. In the present embodiment, the oscillating mechanism is a translating mechanism 25, for example a rotary shaft 29 moving in a guide 28 coupled to a driving means (e.g. a motor not shown). The translating support roller 34 is driven to rotates around the rotary shaft 29 that is driven to oscillate by translation into the guide 28 over the defined distance 16 from the engaged position P1 (FIG. 21) to the remote position P2 (FIG. 22). Similarly to the third embodiment, the distance 16 is defined by the extreme engaged and remote positions P1 and P2, respectively. When the translating support roller 34 is in the engaged position P1 relatively to the transfer roller 2, the transfer roller 2 applies the coating material 21 to the web of absorbent substrate 20 at the contacting location. In the engaged position P1, the sequence of contact between the chamber doctor blade 30, the dosing roller 1, the transfer roller 2, the running web of absorbent substrate 20 and the translating support roller 34 results in applying sufficiently and uniformly the coating material 21 to the web 20. It is the duration of the engaged position P1 that determines the coating length L where the coating material 21 is applied to the web of absorbent substrate 20.

In the fifth, sixth and seventh embodiments, the chamber doctor blade 30 may be replaced by a spraying unit or a slot dye (not shown).

In all the hereinbefore described embodiments, alternatives or variants, the dosing roller 1 may be an anilox roller.

In all the hereinbefore described embodiments, alternatives or variants, the support roller may be replaced by a support plate as usual in the field of converting web of absorbent substrate, in particular web of tissue paper into hygiene product like e.g. toilet paper rolls.

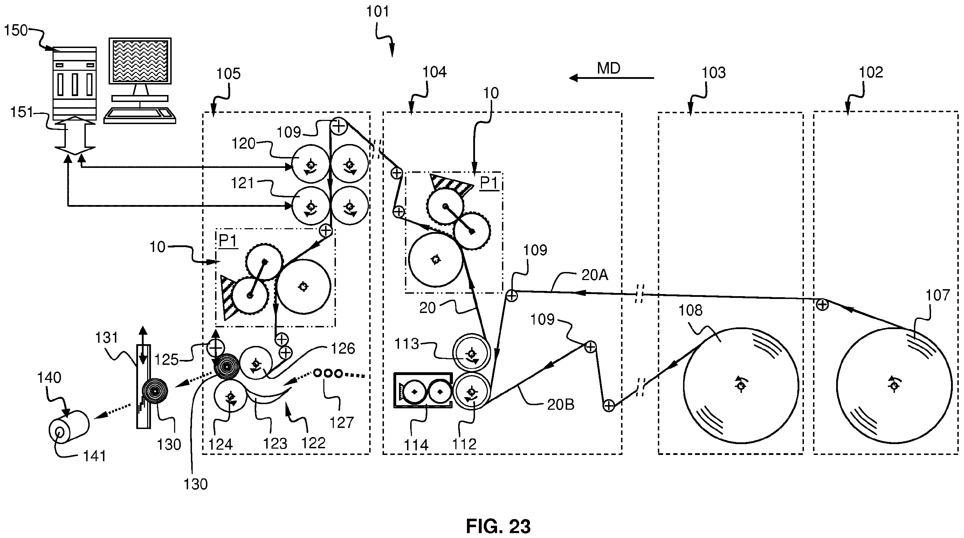

FIG. 23 is a side view schematically illustrating a converting machine/line 101 including an apparatus for coating a web of absorbent substrate 10 of the invention. In this exemplary converting machine/line 101, the apparatus for coating a web of absorbent substrate 10 according to the first embodiment in the engaged position P1 is depicted. However, a converting machine/line 101 including an apparatus for coating a web of absorbent substrate 10 according to anyone of the other embodiment is also possible.

In this example, the converting machine/line 101 includes two unwinding units 102 and 103, an embossing unit 104, and the rewinding machine 105.

More precisely, at the stage of FIG. 23, absorbent log base webs 20A, 20B have already been produced according to a known papermaking process. FIG. 23 illustrates a later stage which is a stage where a converting process takes place. The converting process converts large parent log base webs 20A, 20B (e.g. having a strip width from around 1,80 m to around 7 m) into retail sized rolls 140, e.g. bathroom tissue rolls, paper towels rolls (e.g. having a strip width from around 8 cm to around 40 cm). In this example, the converting machine/line 101 produces retail sized rolls having two plies.

A first unwinding unit 102 provides a first absorbent log base web 20A from a first parent roll 107. A second unwinding unit 103 provides a second absorbent log base web 20B from a second parent roll 108. Both absorbent log base webs 20A, 20B are fed to the embossing unit 104.

Various rollers 109 are appropriately positioned in order to control the path of the absorbent log base webs 20A, 20B along the converting machine/line 101, within and between the various units 102, 103, 104, 105. The absorbent log base webs 20A, 20B travels into the converting machine/line 101 according to the machine direction MD from the unwinding units 102 and 103, towards the embossing unit 104, and towards the rewinding machine 105.

The embossing unit 104 includes an engraved cylinder 112, a mating rubber cylinder 113, both rotating in opposite directions, and a glue dispenser 114. The absorbent log base webs 20A, 20B are superposed and combined into the embossing unit 104 in order to form a web of absorbent substrate 20. The engraved cylinder 112 may be engraved with a microstructure pattern combining various embossing tips (not shown). The engraved cylinder 112 may perform a simple or a double-level engraving into the superposed absorbent log base webs 20A, 20B. The glue dispenser 114 typically includes a vat, an applicator cylinder and a dipping cylinder. The applicator cylinder abuts the superposed absorbent log base webs 20A, 20B against the engraved cylinder 112. The dipping cylinder picks up the adhesive in the vat and transfers the adhesive to the applicator cylinder. The applicator cylinder is arranged to exercise a determined pressure on the engraved cylinder at the distal area of protuberances of the embossed absorbent log base webs 20A, 20B. At said determined pressure, the adhesive crosses through both absorbent log base webs 20A, 20B. This is used to combine both webs and, also, to emboss or micro-emboss at least one of the absorbent log base webs 20A, 20B in order to generate esthetical effects or modify the thickness, or the softness, or the suppleness of the resulting web of absorbent substrate 20.

The rewinding machine 105 includes a perforating module 120, a cutting module 121, a winding module 122 and a control module 150. The rewinding machine 105 winds the web of absorbent substrate 20 into multiple logs 130.

The perforating module 120 is arranged to provide the web of absorbent substrate 20 with regularly spaced perforation lines substantially transversally orientated relatively to the machine direction MD (i.e. the perforation lines are substantially orientated according to the cross-machine direction).

The cutting module 121 is arranged to sever the web of absorbent substrate 20 substantially transversally relatively to the machine direction (i.e. the separation line is substantially orientated according to the cross-machine direction). The severing of web occurs at a transition phase, namely when a first log is finished at the end of a log production cycle, and before a second subsequent log starts to be wound at the beginning of a new log production cycle.

The winding module 122 is arranged to wind the web of absorbent substrate 20 so as to produce logs 130 of web of absorbent substrate. For example, the winding module 122 is of the peripheral or the surface type. The winding module 122 includes a rolling surface 123, a first winding roller 124, a second winding roller 125, a third winding roller 126 and a core supplier 127. The log 130 is formed by winding the web of absorbent substrate 20 onto a core 141. Cores 141 are sequentially provided by the core supplier 127 through the rolling surface 123 before the beginning of a new log production cycle. The log 130 is maintained in position during the winding by the first, second and third winding rollers 124, 125, 126 rotating in surface contact with the log 130. One of the winding rollers 124, 125, 126 imposes the rotation movement of the log 130.

The control module 150 is coupled to the perforating module 120 and to the cutting module 121 by means of an interface 151. The control module 150 controls the operation of the perforating module 120 and the cutting module 121 in an independent manner. For example, the control module 150 deactivates the perforating module 120 and activates the cutting module 121 at a transition phase between two consecutive logs. The control module 150 activates the perforating module 120 and deactivates the cutting module 121 out of transition phases. As an example, the perforating module 120 operates during almost around 99,6% of the log production cycle, while the cutting module 121 operates during only around 0,4% of the log production cycle.

The produced log 130 is then cut by multiple log saws 131 (only one is visible) into multiple and individual rolls 140 of absorbent material sheet (only one is shown).

As an example, the illustrated converting machine/line 101 shows two possible locations of the absorbent substrate web coating apparatus 10. One absorbent substrate web coating apparatus 10 is shown located proximate to the embossing unit 104, the other absorbent substrate web coating apparatus 10 is located proximate to and upstream of the rewinding unit 105. These are only non limiting examples. It can be understood that a converting machine/line 101 may include only one absorbent substrate web coating apparatus 10 located within the unit that is the most appropriate to perform a coating task.

The drawings and their descriptions hereinbefore illustrate rather than limit the invention.

Though the invention has been described with respect to various examples of oscillating mechanism, either rotating or translating, using arm, shaft, guide, etc... these are not limitative examples. The skilled person will readily recognize that, in practice, there exist other ways of realizing oscillating mechanism.

Further, though the drawings show a particular positioning of the different rollers relatively to each other in the coating apparatus, this is a mere example because the rollers can be positioned vertically or a combination of horizontal/vertical position. Also, the web of absorbent substrate appearing to be depicted and described as including a single ply is only an example as the invention would be applicable to web of absorbent substrate including two plies, three plies or more. Furthermore, the coating apparatus may be used to coat the web of absorbent substrate irrespective of the side of the web of absorbent substrate, namely either the bottom side, or the top side, or between plies when the web of absorbent substrate includes more than two plies.

## Claims

1. An absorbent substrate web coating apparatus (10) for applying a coating material (21) on a web of absorbent substrate (20) over a defined length (L) including:
- a support device (3, 33, 34) arranged to support the web of absorbent substrate (20) running at a speed ranging from around 200 m/min to around 1.200°m/min,
- a dosing and transfer device (1, 2) arranged to cooperate with a coating material storing and transferring unit (30, 31, 32) such as to continuously coat a surface (9) of the dosing and transfer device (1) with a uniform quantity of coating material (21), and
- an oscillating mechanism (5, 15, 25, 35) arranged to oscillate the dosing and transfer device (1, 2) or the support device (33, 34) from an engaged position (P1) to a remote position (P2),
wherein the dosing and transfer device (1, 2) is arranged relatively to the support device (3, 33, 34) such that the transfer device (1, 2) applies the coating material (21) to the web of absorbent substrate (20) at a contacting location where the web of absorbent material (20) contacts the support device (3, 33, 34) and the dosing and transfer device (1, 2) only in the engaged position (P1).

2. The absorbent substrate web coating apparatus of claim 1, wherein
- the support device is a support roller (3, 33, 34) or a support plate, and
- the dosing and transfer device comprises a transfer roller (2).

3. The absorbent substrate web coating apparatus of claim 2, wherein the dosing and transfer device further comprise a dosing roller (1), the dosing roller (1) being arranged to cooperate with the coating material storing and transferring unit (30, 31, 32) such as to continuously coat the surface (9) of the dosing roller (1) with an uniform quantity of coating material (21), and the transfer roller (2) being coupled to the dosing roller (1) such as to transfer the coating material (21) from the dosing roller (1) to the transfer roller (2).

4. The absorbent substrate web coating apparatus of claim 3, wherein the oscillating mechanism includes a rotating mechanism (5) arranged to cause the transfer roller (2) to rotate around a circumference (9) of the dosing roller (1) over an angular sector (6) defined by the engaged (P1) and remote (P2) position and centered on a rotary shaft (7) of the dosing roller (1).

5. The absorbent substrate web coating apparatus of claim 3, wherein the oscillating mechanism includes a translating mechanism (15) arranged to cause the dosing roller (1) and the transfer roller (2) to translate together over a distance (16) defined by the engaged position (P1) and remote (P2) position.

6. The absorbent substrate web coating apparatus according to anyone of the claims 2 to 5, wherein an additional transfer roller (4) positioned between the support roller (3, 33, 34) and the transfer roller (2) is permanently contacting the support roller (3) whatever the position of the transfer roller (2).

7. The absorbent substrate web coating apparatus of claim 2, wherein the support roller is an eccentric roller (33).

8. The absorbent substrate web coating apparatus of claim 2, wherein the oscillating mechanism includes another rotating mechanism (35) comprising an oscillating arm (38) coupled at one end to a rotary shaft (29) of the support roller (34), and at another end to a pivot (37), the rotating mechanism (35) being arranged to cause the support roller (34) to rotate over an angular sector (36) defined by the engaged (P1) and remote position (P2) and centered on the pivot (37).

9. The absorbent substrate web coating apparatus of claim 2, wherein the oscillating mechanism includes another translating mechanism (25) arranged to cause the support roller (34) to translate over a distance (16) defined by the engaged (P1) and remote (P2) position.

10. The absorbent substrate web coating apparatus according to anyone of the claims 1 to 9, wherein the coating material storing and transferring unit is chosen from the group consisting of a chamber doctor blade (30), a spraying unit (31), and a slot dye (32).

11. A converting machine (101) comprising an embossing unit (104) and/or a rewinding unit (105) wherein the converting machine (101) further comprises an absorbent substrate web coating apparatus (10) according to anyone of the claims 1 to 10 located proximate to the embossing unit (104) and/or located proximate to and upstream of the rewinding unit (105).

12. Use of the converting machine of claim 11 for manufacturing absorbent sheet products chosen among the group comprising napkins, towels, kitchen towels, hand towels, toilet papers, wipes and facial tissues.

13. An absorbent substrate web coating method for applying a coating material (21) on a web of absorbent substrate (20) over a defined length (L) comprising:
- conveying the web of absorbent substrate (20) at a speed ranging from around 200 m/min to around 1.200°m/min and supporting the conveyed web of absorbent substrate (20) by a support device (3, 33, 34),
- continuously coating a surface (9) of a dosing and transfer device (1, 2) with an uniform quantity of coating material (21) by a coating material storing and transferring unit (30, 31, 32) cooperating with the dosing and transfer device (1, 2),
- oscillating the dosing and transfer device (1, 2) or the support device (33, 34) from an engaged position (P1) to a remote position (P2), and
- applying the coating material (21) to the web of absorbent substrate (20) at a contacting location where the web of absorbent substrate (20) contacts the support device (3, 33, 34) and the dosing and transfer device (1, 2) only in the engaged position (P1).

14. The absorbent substrate web coating method of claim 13, wherein the dosing and transfer device comprises a transfer roller (2), and the oscillating step includes rotating the transfer roller (2) over an angular sector (6) defined by the engaged position (P1) and the remote position (P2).

15. The absorbent substrate web coating method of claim 13, wherein the dosing and transfer device comprises a transfer roller (2), and the oscillating step includes translating the transfer roller (2) over a distance (16) defined by the engaged position (P1) and remote position (P2).

16. The absorbent substrate web coating method of claim 13, wherein the support device is a support roller (3, 33, 34), and the oscillating step includes eccentrically rotating the support roller (33), the engaged position (P1) corresponding to a defined angular sector (26) of the support roller (33).

17. The absorbent substrate web coating method of claim 13, wherein the support device is a support roller (3, 33, 34), and the oscillating step includes rotating the support roller (33) over an angular sector (26, 36) defined by the engaged position (P1) and remote position (P2).

18. The absorbent substrate web coating method of claim 13, wherein the support device is a support roller (3, 33, 34), and the oscillating step includes translating the support roller (34) over a distance (16) defined by the engaged position (P1) and remote position (P2).

## Patentansprüche

1. Beschichtungsvorrichtung (10) für eine Bahn aus einem saugfähigen Substrat zum Auftragen eines Beschichtungsmaterials (21) auf eine Bahn aus einem saugfähigen Substrat (20) über eine definierte Länge (L), umfassend:
- eine Trägervorrichtung (3, 33, 34), die so angeordnet ist, dass sie die Bahn aus saugfähigem Substrat (20), die mit einer Geschwindigkeit im Bereich von etwa 200 m/min bis etwa 1200 m/min läuft, tragen kann;
- eine Dosier- und Übertragungsvorrichtung (1, 2), die so angeordnet ist, dass sie mit einer Beschichtungsmateriallager- und -übertragungseinheit (30, 31, 32) so zusammenwirken kann, dass eine Oberfläche (9) der Dosier- und Übertragungsvorrichtung (1) kontinuierlich mit einer gleichmäßigen Menge Beschichtungsmaterial (21) beschichtet wird; und
- einen Schwingmechanismus (5, 15, 25, 35), der so angeordnet ist, dass er die Dosier- und Übertragungsvorrichtung (1, 2) oder die Trägervorrichtung (33, 34) von einer eingerasteten Position (P1) aus zu einer entfernten Position (P2) schwingen lassen kann;
wobei die Dosier- und Übertragungsvorrichtung (1, 2) relativ zu der Trägervorrichtung (3, 33, 34) so angeordnet ist, dass die Übertragungsvorrichtung (1, 2) das Beschichtungsmaterial (21) an einem Kontaktort, wo die Bahn aus saugfähigem Substrat (20) die Trägervorrichtung (3, 33, 34) und die Dosier- und Übertragungsvorrichtung (1, 2) nur in der eingerasteten Position (P1) berührt, auf die Bahn aus saugfähigem Substrat (20) aufträgt.

2. Beschichtungsvorrichtung für eine Bahn aus einem saugfähigen Substrat gemäß Anspruch 1, wobei
- die Trägervorrichtung eine Trägerwalze (3, 33, 34) oder eine Trägerplatte ist und
- die Dosier- und Übertragungsvorrichtung eine Übertragungswalze umfasst.

3. Beschichtungsvorrichtung für eine Bahn aus einem saugfähigen Substrat gemäß Anspruch 2, wobei die Dosier- und Übertragungsvorrichtung weiterhin eine Dosierwalze (1) umfasst, wobei die Dosierwalze (1) so angeordnet ist, dass sie mit der Beschichtungsmateriallager- und -übertragungseinheit (30, 31, 32) so zusammenwirken kann, dass die Oberfläche (9) der Dosierwalze (1) kontinuierlich mit einer gleichmäßigen Menge Beschichtungsmaterial (21) beschichtet wird, und die Übertragungswalze (2) so an die Dosierwalze (1) gekoppelt ist, dass das Beschichtungsmaterial (21) von der Dosierwalze (1) auf die Übertragungswalze (2) übertragen wird.

4. Beschichtungsvorrichtung für eine Bahn aus einem saugfähigen Substrat gemäß Anspruch 3, wobei der Schwingmechanismus einen Rotationsmechanismus (5) umfasst, der so angeordnet ist, dass er bewirken kann, dass die Übertragungswalze (2) über einen Winkelsektor (6), der durch die eingerastete (P1) und die entfernte (P2) Position definiert ist und auf einer Rotationswelle (7) der Dosierwalze (1) zentriert ist, um einen Umfang (9) der Dosierwalze (1) herum schwenkt.

5. Beschichtungsvorrichtung für eine Bahn aus einem saugfähigen Substrat gemäß Anspruch 3, wobei der Schwingmechanismus einen Translationsmechanismus (15) umfasst, der so angeordnet ist, dass er bewirken kann, dass die Dosierwalze (1) und die Übertragungswalze (2) zusammen über eine Strecke (16), die durch die eingerastete (P1) und die entfernte (P2) Position definiert ist, verschoben werden.

6. Beschichtungsvorrichtung für eine Bahn aus einem saugfähigen Substrat gemäß einem der Ansprüche 2 bis 5, wobei eine zusätzliche Übertragungswalze (4), die sich zwischen der Trägerwalze (3, 33, 34) und der Übertragungswalze (2) befindet, unabhängig von der Position der Übertragungswalze (2) die Trägerwalze (3) permanent berührt.

7. Beschichtungsvorrichtung für eine Bahn aus einem saugfähigen Substrat gemäß Anspruch 2, wobei die Trägerwalze eine exzentrische Walze (33) ist.

8. Beschichtungsvorrichtung für eine Bahn aus einem saugfähigen Substrat gemäß Anspruch 2, wobei der Schwingmechanismus einen weiteren Rotationsmechanismus (35) umfasst, der einen Schwingarm (38) umfasst, der an einem Ende an eine Rotationswelle (29) der Trägerwalze (34) und an einem anderen Ende an einen Drehpunkt (37) gekoppelt ist, wobei der Rotationsmechanismus (35) so angeordnet ist, dass er bewirken kann, dass die Trägerwalze (34) über einen Winkelsektor (36) schwenkt, der durch die eingerastete (P1) und die entfernte (P2) Position definiert ist und auf dem Drehpunkt (37) zentriert ist.

9. Beschichtungsvorrichtung für eine Bahn aus einem saugfähigen Substrat gemäß Anspruch 2, wobei der Schwingmechanismus einen weiteren Translationsmechanismus (25) umfasst, der so angeordnet ist, dass er bewirken kann, dass die Trägerwalze (34) über eine Strecke (16), die durch die eingerastete (P1) und die entfernte (P2) Position definiert ist, verschoben wird.

10. Beschichtungsvorrichtung für eine Bahn aus einem saugfähigen Substrat gemäß einem der Ansprüche 1 bis 9, wobei die Beschichtungsmateriallager- und -übertragungseinheit aus der Gruppe ausgewählt ist, die aus einer Kammerrakel (30), einer Sprüheinheit (31) und einer Schlitzdüse (32) besteht.

11. Verarbeitungsmaschine (101), die eine Prägeeinheit (104) und/oder eine Umspulanlage (105) umfasst, wobei die Verarbeitungsmaschine (101) weiterhin eine Beschichtungsvorrichtung für eine Bahn aus einem saugfähigen Substrat gemäß einem der Ansprüche 1 bis 10 umfasst, die sich in der Nähe der Prägeeinheit (104) befindet und/oder sich in der Nähe der Umspulanlage (105) und derselben vorgeschaltet befindet.

12. Verwendung der Verarbeitungsmaschine gemäß Anspruch 11 zur Herstellung von saugfähigen Flächenprodukten, die aus der Gruppe ausgewählt sind, die Windeln, Tücher, Küchentücher, Handtücher, Toilettenpapier, Wischtücher und Kosmetiktücher umfasst.

13. Beschichtungsverfahren für eine Bahn aus einem saugfähigen Substrat zum Auftragen eines Beschichtungsmaterials (21) auf eine Bahn aus einem saugfähigen Substrat (20) über eine definierte Länge (L), umfassend:
- Transportieren der Bahn aus saugfähigem Substrat (20) mit einer Geschwindigkeit im Bereich von etwa 200 m/min bis etwa 1200 m/min und Tragen der transportierten Bahn aus saugfähigem Substrat (20) durch eine Trägervorrichtung (3, 33, 34);
- kontinuierliches Beschichten einer Oberfläche (9) einer Dosier- und Übertragungsvorrichtung (1, 2) mit einer gleichmäßigen Menge Beschichtungsmaterial (21) durch eine Beschichtungsmateriallager- und -übertragungseinheit (30, 31, 32), die mit der Dosier- und Übertragungsvorrichtung (1, 2) zusammenwirkt;
- Schwingenlassen der Dosier- und Übertragungsvorrichtung (1, 2) oder der Trägervorrichtung (33, 34) von einer eingerasteten Position (P1) aus zu einer entfernten Position (P2); und
- Auftragen des Beschichtungsmaterials (21) auf die Bahn aus saugfähigem Substrat (20) an einem Kontaktort, wo die Bahn aus saugfähigem Substrat (20) die Trägervorrichtung (3, 33, 34) und die Dosier- und Übertragungsvorrichtung (1, 2) nur in der eingerasteten Position (P1) berührt.

14. Beschichtungsverfahren für eine Bahn aus einem saugfähigen Substrat gemäß Anspruch 13, wobei die Dosier- und Übertragungsvorrichtung eine Übertragungswalze (2) umfasst und der Schritt des Schwingenlassens das Schwenken der Übertragungswalze (2) über einen Winkelsektor (6), der durch die eingerastete (P1) und die entfernte (P2) Position definiert ist, umfasst.

15. Beschichtungsverfahren für eine Bahn aus einem saugfähigen Substrat gemäß Anspruch 13, wobei die Dosier- und Übertragungsvorrichtung eine Übertragungswalze (2) umfasst und der Schritt des Schwingenlassens das Verschieben der Übertragungswalze (2) über eine Strecke (16), die durch die eingerastete (P1) und die entfernte (P2) Position definiert ist, umfasst.

16. Beschichtungsverfahren für eine Bahn aus einem saugfähigen Substrat gemäß Anspruch 13, wobei die Trägervorrichtung eine Trägerwalze (3, 33, 34) ist und der Schritt des Schwingenlassens das exzentrische Schwenken der Trägerwalze (33) umfasst, wobei die eingerastete Position (P1) einem definierten Winkelsektor (26) der Trägerwalze (33) entspricht.

17. Beschichtungsverfahren für eine Bahn aus einem saugfähigen Substrat gemäß Anspruch 13, wobei die Trägervorrichtung eine Trägerwalze (3, 33, 34) ist und der Schritt des Schwingenlassens das Schwenken der Trägerwalze (33) über einen Winkelsektor (26, 36), der durch die eingerastete (P1) und die entfernte (P2) Position definiert ist, umfasst.

18. Beschichtungsverfahren für eine Bahn aus einem saugfähigen Substrat gemäß Anspruch 13, wobei die Trägervorrichtung eine Trägerwalze (3, 33, 34) ist und der Schritt des Schwingenlassens das Verschieben der Trägerwalze (34) über eine Strecke (16), die durch die eingerastete (P1) und die entfernte (P2) Position definiert ist, umfasst.

## Revendications

1. Appareil de revêtement de bande de substrat absorbant (10) pour appliquer un matériau de revêtement (21) sur une bande de substrat absorbant (20) sur une longueur définie (L), comprenant :
- un dispositif de support (3, 33, 34) agencé pour supporter la bande de substrat absorbant (20) se déplaçant à une vitesse comprise dans la plage allant d'environ 200 m/min à environ 1200 m/min,
- un dispositif de dosage et de transfert (1, 2) agencé pour coopérer avec une unité de stockage et de transfert de matériau de revêtement (30, 31, 32) de façon à revêtir en continu une surface (9) du dispositif de dosage et de transfert (1) avec une quantité uniforme de matériau de revêtement (21), et
- un mécanisme d'oscillation (5, 15, 25, 35) agencé pour faire osciller le dispositif de dosage et de transfert (1, 2) ou le dispositif de support (33, 34) d'une position engagée (P1) à une position à distance (P2),
le dispositif de dosage et de transfert (1, 2) étant disposé par rapport au dispositif de support (3, 33, 34) de telle sorte que le dispositif de transfert (1, 2) applique le matériau de revêtement (21) sur la bande de substrat absorbant (20) à un emplacement de contact où la bande de matériau absorbant (20) entre en contact avec le dispositif de support (3, 33, 34) et le dispositif de dosage et de transfert (1, 2) uniquement dans la position engagée (P1).

2. Appareil de revêtement de bande de substrat absorbant selon la revendication 1, dans lequel
- le dispositif de support est un rouleau de support (3, 33, 34) ou une plaque de support, et
- le dispositif de dosage et de transfert comprend un rouleau de transfert (2).

3. Appareil de revêtement de bande de substrat absorbant selon la revendication 2, dans lequel le dispositif de dosage et de transfert comprend en outre un rouleau de dosage (1), le rouleau de dosage (1) étant agencé pour coopérer avec l'unité de stockage et de transfert de matériau de revêtement (30, 31, 32) de façon à revêtir en continu la surface (9) du rouleau de dosage (1) avec une quantité uniforme de matériau de revêtement (21), et le rouleau de transfert (2) étant couplé au rouleau de dosage (1) de façon à transférer le matériau de revêtement (21) du rouleau de dosage (1) au rouleau de transfert (2).

4. Appareil de revêtement de bande de substrat absorbant selon la revendication 3, dans lequel le mécanisme d'oscillation comprend un mécanisme de rotation (5) agencé pour amener le rouleau de transfert (2) à tourner autour d'une circonférence (9) du rouleau de dosage (1) sur un secteur angulaire (6) défini par les positions engagée (P1) et à distance (P2) et centré sur un arbre rotatif (7) du rouleau de dosage (1).

5. Appareil de revêtement de bande de substrat absorbant selon la revendication 3, dans lequel le mécanisme d'oscillation comprend un mécanisme de translation (15) agencé pour amener le rouleau de dosage (1) et le rouleau de transfert (2) à se déplacer conjointement en translation sur une distance (16) définie par la position engagée (P1) et la position à distance (P2) .

6. Appareil de revêtement de bande de substrat absorbant selon l'une quelconque des revendications 2 à 5, dans lequel un rouleau de transfert supplémentaire (4) positionné entre le rouleau de support (3, 33, 34) et le rouleau de transfert (2) est en contact permanent avec le rouleau de support (3) quelle que soit la position du rouleau de transfert (2).

7. Appareil de revêtement de bande de substrat absorbant selon la revendication 2, dans lequel le rouleau de support est un rouleau excentrique (33).

8. Appareil de revêtement de bande de substrat absorbant selon la revendication 2, dans lequel le mécanisme d'oscillation comprend un autre mécanisme de rotation (35) comprenant un bras oscillant (38) couplé, au niveau d'une extrémité, à un arbre rotatif (29) du rouleau de support (34) et, au niveau d'une autre extrémité, à un pivot (37), le mécanisme de rotation (35) étant agencé pour amener le rouleau de support (34) à tourner sur un secteur angulaire (36) défini par les positions engagée (P1) et à distance (P2) et centré sur le pivot (37).

9. Appareil de revêtement de bande de substrat absorbant selon la revendication 2, dans lequel le mécanisme d'oscillation comprend un autre mécanisme de translation (25) agencé pour amener le rouleau de support (34) à se déplacer en translation sur une distance (16) définie par les positions engagée (P1) et à distance (P2).

10. Appareil de revêtement de bande de substrat absorbant selon l'une quelconque des revendications 1 à 9, dans lequel l'unité de stockage et de transfert de matériau de revêtement est choisie parmi le groupe constitué d'une racle de compartiment (30), d'une unité de pulvérisation (31) et d'une filière plate (32).

11. Machine de transformation (101) comprenant une unité de gaufrage (104) et/ou une unité de rembobinage (105), la machine de transformation (101) comprenant en outre un appareil de revêtement de bande de substrat absorbant (10) selon l'une quelconque des revendications 1 à 10 situé à proximité de l'unité de gaufrage (104) et/ou situé à proximité et en amont de l'unité de rembobinage (105) .

12. Utilisation de la machine de transformation selon la revendication 11 pour fabriquer des produits en feuille absorbants choisis parmi le groupe comprenant des serviettes de table, des serviettes de toilette, des essuie-touts, des essuie-mains, du papier toilette, des lingettes et des mouchoirs.

13. Procédé de revêtement de bande de substrat absorbant pour appliquer un matériau de revêtement (21) sur une bande de substrat absorbant (20) sur une longueur définie (L), comprenant :
- transporter la bande de matériau absorbant (20) à une vitesse comprise dans la plage allant d'environ 200 m/min à environ 1200 m/min, et supporter la bande de substrat absorbant (20), transportée, par un dispositif de support (3, 33, 34),
- revêtir en continu une surface (9) d'un dispositif de dosage et de transfert (1, 2) avec une quantité uniforme de matériau de revêtement (21) par une unité de stockage et de transfert de matériau de revêtement (30, 31, 32) coopérant avec le dispositif de dosage et de transfert (1, 2),
- faire osciller le dispositif de dosage et de transfert (1, 2) ou le dispositif de support (33, 34) d'une position engagée (P1) à une position à distance (P2), et
- appliquer le matériau de revêtement (21) sur la bande de substrat absorbant (20) à un emplacement de contact où la bande de substrat absorbant (20) entre en contact avec le dispositif de support (3, 33, 34) et le dispositif de dosage et de transfert (1, 2) uniquement dans la position engagée (P1).

14. Procédé de revêtement de bande de substrat absorbant selon la revendication 13, dans lequel le dispositif de dosage et de transfert comprend un rouleau de transfert (2), et l'étape d'oscillation comprend faire tourner le rouleau de transfert (2) sur un secteur angulaire (6) défini par la position engagée (P1) et la position à distance (P2).

15. Procédé de revêtement de bande de substrat absorbant selon la revendication 13, dans lequel le dispositif de dosage et de transfert comprend un rouleau de transfert (2), et l'étape d'oscillation comprend déplacer en translation le rouleau de transfert (2) sur une distance (16) définie par la position engagée (P1) et la position à distance (P2).

16. Procédé de revêtement de bande de substrat absorbant selon la revendication 13, dans lequel le dispositif de support est un rouleau de support (3, 33, 34), et l'étape d'oscillation comprend faire tourner de manière excentrique le rouleau de support (33), la position engagée (P1) correspondant à un secteur angulaire défini (26) du rouleau de support (33).

17. Procédé de revêtement de bande de substrat absorbant selon la revendication 13, dans lequel le dispositif de support est un rouleau de support (3, 33, 34), et l'étape d'oscillation comprend faire tourner le rouleau de support (33) sur un secteur angulaire (26, 36) défini par la position engagée (P1) et la position à distance (P2).

18. Procédé de revêtement de bande de substrat absorbant selon la revendication 13, dans lequel le dispositif de support est un rouleau de support (3, 33, 34), et l'étape d'oscillation comprend déplacer en translation le rouleau de support (34) sur une distance (16) définie par la position engagée (P1) et la position à distance (P2).
